# EUROPEAN PATENT APPLICATION

(11) **EP 2 421 041 A1**
(43) Date of publication of application: **22.02.2012**
(21) Application number: 10764347.0
(22) Date of filing: 26.03.2010
(51) Int. Cl.: H01L 27/14, H01L 23/02, H01L 23/04

(54) **SEMICONDUCTOR DEVICE AND METHOD FOR MANUFACTURING SEMICONDUCTOR DEVICE**

(30) Priority: 15.04.2009 JP 2009099206
(71) Applicant: Olympus Medical Systems Corp., Tokyo 151-0072 (JP)
(72) Inventor: HOSHI Kazuhisa, Tokyo 151-0072 (JP); HIROYA, Jun, Tokyo 151-0072 (JP); IWASAKI, SEIJI, Tokyo 151-0072 (JP); MURAMATSU, Akira, Tokyo 151-0072 (JP); WATAYA, Yuichi, Tokyo 151-0072 (JP); KUCHIMARU, Toru, Tokyo 151-0072 (JP); ISHII, Hiroshi, Tokyo 151-0072 (JP); YAMASHITA, Tomoaki, Tokyo 151-0072 (JP)
(74) Representative: Gunzelmann, Rainer
(86) International application number: PCT/JP2010/055416
(87) International publication number: WO 2010/119762

(57) **Abstract**

There is provided a semiconductor device including a bare chip 10, wherein at least two or more groups of external connection terminals 21 and 22 to which a substrate that drives the bare chip 10 by inputting a signal from an external apparatus to the bare chip 10 is electrically connected are formed outside an image area 11 of the bare chip 10.

## Description

### Technical Field

The present invention relates to a semiconductor device including a semiconductor chip and a method for manufacturing the semiconductor device.

### Background Art

An electronic endoscope, a mobile phone with a camera, a digital camera, and the like including a semiconductor device such as a solid-state image pickup apparatus provided with an image sensor (e.g., a CCD or a CMOS) have been well known.

Among modem solid-state image pickup apparatuses, ones of wafer level chip size package (hereinafter referred to as WL-CSP) type are well known.

For WL-CSPs, a technique is known for separating a sensor wafer including a plurality of semiconductor chips, each having an image sensor, into the chips by, e.g., dicing and then bonding a cover glass onto each semiconductor chip to complete a plurality of solid-state image pickup apparatus packages (semiconductor packages). Other known techniques include one for bonding a cover glass wafer onto a sensor wafer at the wafer level and then separating the sensor wafer into semiconductor chips by, e.g., dicing to complete a plurality of solid-state image pickup apparatus packages. Such a technique is disclosed in, for example, Japanese Patent Application Laid-Open Publication No. 2006-303481.

If a solid-state image pickup apparatus of the above-described type is used in an endoscope, the solid-state image pickup apparatus is provided inside an insertion portion of the endoscope. Diameters of endoscopes vary according to the type of endoscope.

Accordingly, if solid-state image pickup apparatuses used in endoscopes are to be manufactured, solid-state image pickup apparatuses of, e.g., two sizes need to be manufactured so as to suit the sizes to diameters of insertion portions of the endoscopes. More specifically, image sensors of two sizes need to be separately manufactured. This causes the problem of an increase in complexity of a manufacturing process, in addition to the problems of an increase in development costs and manufacturing control costs.

Note that examples of solid-state image pickup apparatuses of two sizes are a solid-state image pickup apparatus packaged to be relatively large and include a general-purpose external terminal and a solid-state image pickup apparatus packaged to be smaller than the general-purpose solid-state image pickup apparatus.

The present invention has been made in consideration of the circumstances, and has as an object to provide a semiconductor device with a configuration which allows a semiconductor chip or a semiconductor package to be formed to have either one of the two sizes without changing a size of an image sensor and a method for manufacturing the semiconductor device.

### Disclosure of Invention

### Means for Solving the Problem

A semiconductor device according to the present invention is a semiconductor device including a semiconductor chip, wherein at least two or more groups of external connection terminals to which a substrate that drives the semiconductor chip by inputting a signal from an external apparatus to the semiconductor chip is electrically connected are formed in a region outside an active region of the semiconductor chip.

Another semiconductor device is a semiconductor device including a semiconductor package in which a semiconductor chip is stored in a storage portion, wherein a cutoff region for cutting off a region which does not include the semiconductor chip along a thickness direction of the semiconductor package is provided in the semiconductor package.

A method for manufacturing a semiconductor device according to the present invention is a method for manufacturing a semiconductor device including a semiconductor chip, including an inspection step of electrically connecting a substrate for inspection to a group of terminals for inspection constituted by at least one group of terminals among at least two or more groups of external connection terminals formed in a region outside an active region of the semiconductor chip and performing a driving inspection of the semiconductor chip by inputting a signal from an external apparatus to the semiconductor chip, and a mounting step of electrically connecting a substrate for mounting to a group of terminals for mounting formed in a region which does not overlap with the group of terminals for inspection in a plan view among the external connection terminals after the driving inspection ends.

Another method for manufacturing a semiconductor device according to the present invention is a method for manufacturing a semiconductor device including a semiconductor chip, including a step of electrically connecting a substrate for inspection which doubles as a substrate for mounting to a first group of terminals which doubles as a second group of terminals and is constituted by at least one group of terminals among at least two or more groups of external connection terminals formed in a region outside an active region of the semiconductor chip.

Still another method for manufacturing a semiconductor device according to the present invention is a method for manufacturing a semiconductor device including a semiconductor package in which a semiconductor chip is stored in a storage portion, including performing a cutoff step of cutting off a part of the semiconductor package at a cutoff region provided in a region which does not include the semiconductor chip along a thickness direction of the semiconductor package to form the semiconductor package to be smaller than the semiconductor package before the cutoff.

### Brief Description of the Drawings

Fig. 1 is a plan view showing a sensor wafer including a plurality of semiconductor chips, each of which is to be included in a solid-state image pickup apparatus, according to a first embodiment;
Fig. 2 is a perspective view showing, on an enlarged scale, a semiconductor chip which is separated from the sensor wafer in Fig. 1;
Fig. 3 is a partial perspective view showing a state in which a cover glass is attached to cover an image area in Fig. 2 and a substrate for inspection is mounted on a first group of terminals;
Fig. 4 is a perspective view showing a state in which the first group of terminals is cut off from the semiconductor chip in Fig. 3 and a substrate for mounting is mounted on a second group of terminals;
Figs. 5 are side views of the semiconductor chips with the cover glasses attached in Figs. 3 and 4 as seen from directions V in Figs. 3 and 4;
Fig. 6 is a plan view showing a bare chip used in a solid-state image pickup apparatus according to a second embodiment as seen from a first surface side;
Fig. 7 is a rear view of the bare chip in Fig. 6 as seen from a second surface side;
Fig. 8 is a partial perspective view showing a state in which a cover glass is attached to cover an image area in Fig. 6 and a substrate for inspection is mounted on a first group of terminals;
Fig. 9 is a perspective view showing a state in which a substrate for mounting is mounted on a second group of terminals in Fig. 7;
Fig. 10 is a rear view of a bare chip used in a semiconductor device according to a third embodiment as seen from a back surface side;
Fig. 11 is a partial perspective view showing a state in which a cover glass is attached to cover an image area on a front surface side in Fig. 10 and a substrate for inspection is mounted on a first group of terminals;
Fig. 12 is a perspective view showing a state in which a substrate for mounting is mounted on a second group of terminals in Fig. 10;
Fig. 13 is a plan view showing a semiconductor package in a solid-state image pickup apparatus, according to a fourth embodiment;
Fig. 14 is a sectional view of the semiconductor package taken along line XIV-XIV in Fig. 13;
Fig. 15 is an exploded perspective view showing a state in which parts of the semiconductor package in Fig. 14 are cut off at cutoff regions formed in the semiconductor package;
Fig. 16 is a sectional view showing a semiconductor package in a solid-state image pickup apparatus, according to a fifth embodiment;
Fig. 17 is an exploded perspective view showing a state in which parts of the semiconductor package in Fig. 16 are cut off at cutoff regions formed in the semiconductor package;
Fig. 18 is a sectional view showing a semiconductor package in a solid-state image pickup apparatus, according to a sixth embodiment;
Fig. 19 is an exploded perspective view showing a state in which parts of the semiconductor package in Fig. 18 are cut off at cutoff regions formed in the semiconductor package;
Fig. 20 is a view showing a solid-state image pickup apparatus which is packaged while a substrate for mounting is mounted on the first group of terminals in Fig. 3 or the group second of terminals in Fig. 4; and
Fig. 21 is a sectional view showing an example of an image pickup unit provided with the solid-state image pickup apparatus in Fig. 20.

### Best Mode for Carrying Out the Invention

Embodiments of the present invention will be described below with reference to the drawings. Note that the drawings are schematic and that a relationship between a thickness and a width of members each and a ratio among thicknesses of the members are different from actual ones. Of course, a dimensional relationship and a ratio in one drawing may be different from those in another drawing. A solid-state image pickup apparatus will be described below as an example of a semiconductor device.

### (First Embodiment)

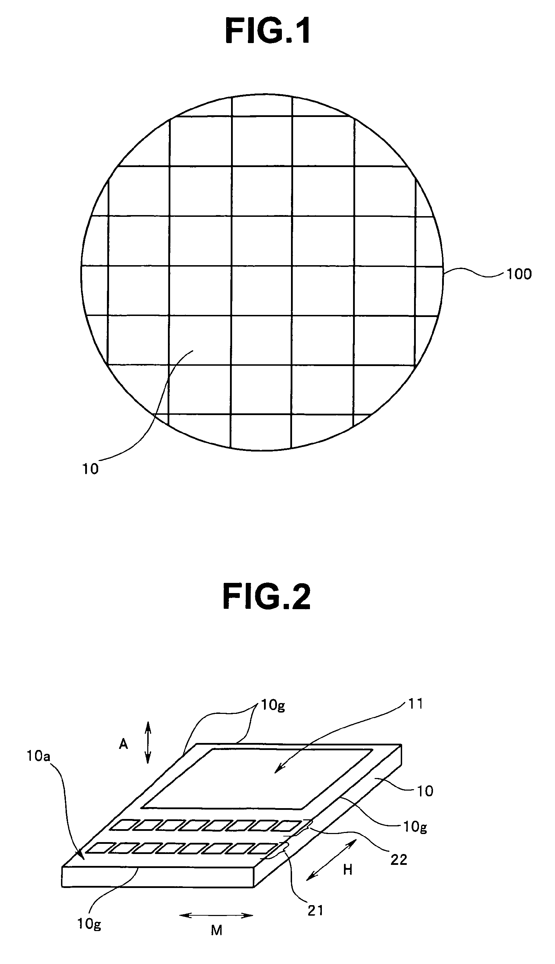

Fig. 1 is a plan view showing a sensor wafer including a plurality of semiconductor chips, each of which is to be included in a solid-state image pickup apparatus, according to the present embodiment. Fig. 2 is a perspective view showing, on an enlarged scale, a semiconductor chip which is separated from the sensor wafer in Fig. 1.

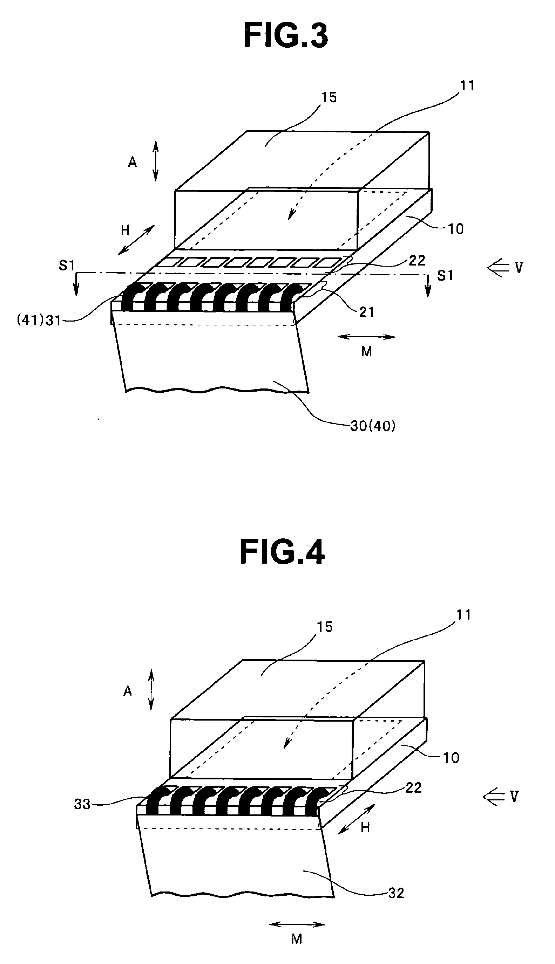

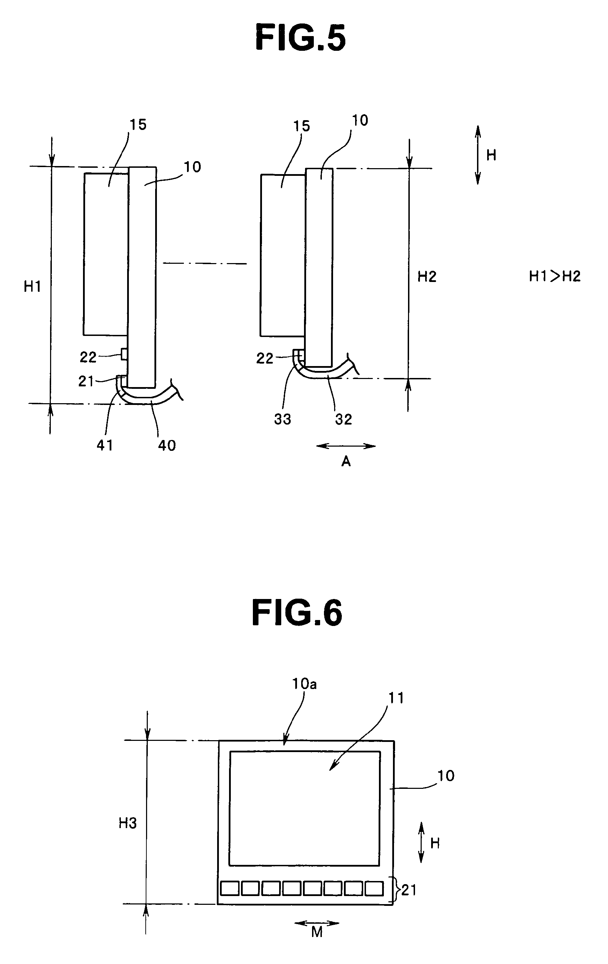

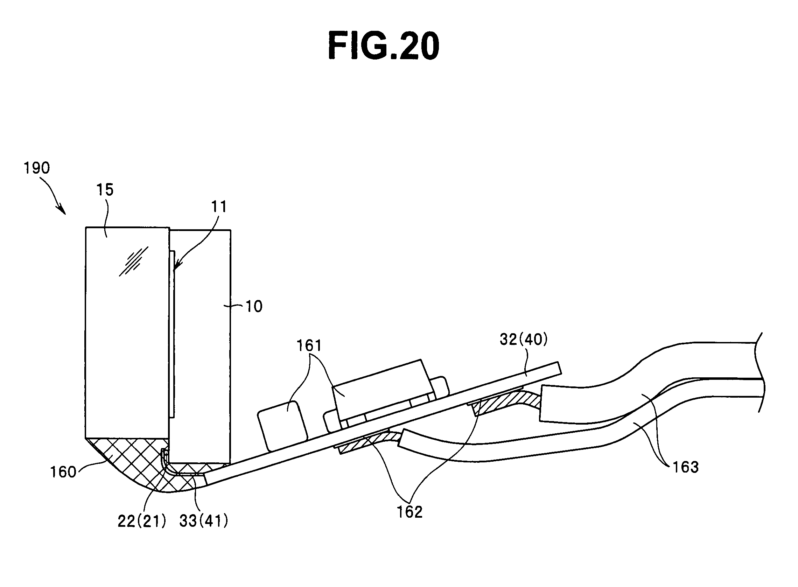

Fig. 3 is a partial perspective view showing a state in which a cover glass is attached to cover an image area in Fig. 2 and a substrate for inspection is mounted on a first group of terminals. Fig. 4 is a perspective view showing a state in which the first group of terminals is cut off from the semiconductor chip in Fig. 3 and a substrate for mounting is mounted on a second group of terminals. Figs. 5 are side views of the semiconductor chips with the cover glasses attached in Figs. 3 and 4 as seen from directions V in Figs. 3 and 4. Fig. 20 is a view showing a solid-state image pickup apparatus which is packaged while a substrate for mounting is mounted on the first group of terminals in Fig. 3 or the second group of terminals in Fig. 4.

As shown in Fig. 2, a semiconductor chip (hereinafter referred to as a bare chip) 10 used in the solid-state image pickup apparatus according to the present embodiment is formed to be rectangular in a plan view from a sensor wafer 100 which includes a plurality of bare chips 10, as shown in Fig. 1, and is made of, e.g., silicon by dicing or the like.

An image area 11 which constitutes an active region and in which an image sensor is provided is formed on a front surface 10a which is a first surface of the bare chip 10. At least two or more groups of external connection terminals 21 and 22 to which substrates 30, 32, and 40 (to be described later) that drive the bare chip 10 by inputting a signal from an external apparatus (not shown) to the bare chip 10 are to be electrically connected are formed in a region outside the image area 11 of the front surface 10a. Note that the external connection terminals 21 and 22 are shown in two groups in Figs. 2 to 4.

More specifically, the external connection terminals 21 constitute a first group of terminals to which an FPC 30 that is a substrate for inspection is electrically connected by wire bonding using wires 31, as shown in Fig. 3. The external connection terminals 22 constitute a second group of terminals to which an FPC 32 that is a substrate for mounting used to mount the bare chip 10 thereon is electrically connected by wire bonding using wires 33, as shown in Fig. 4.

The external connection terminals 21 and 22 are formed along a width direction M of the bare chip 10, as shown in Fig. 2. The external connection terminals 21 are formed at positions which allow only the external connection terminals 21 to be cut off in a thickness direction A along line S1-S1 shown in Fig. 3 in a part closer to an outer edge 10g of the bare chip 10 in a depth direction H of the bare chip 10 than the external connection terminals 22, of the front surface 10a.

Effects of the present invention with the above-described configuration will be described.

When the solid-state image pickup apparatus is to be manufactured, an operator first cuts the plurality of bare chips 10 from the sensor wafer 100 including the plurality of bare chips 10, as shown in Fig. 1, by, e.g., dicing, as shown in Fig. 2.

The operator electrically connects the FPC 30 for inspection to the external connection terminals 21 of each bare chip 10 by wire bonding using the wires 31 and inputs a signal from an external apparatus to the bare chip 10 via the FPC 30, as shown in Fig. 3. With the operation, an inspection step of a driving inspection of the bare chip 10 is performed.

Note that the inspection here is an inspection in the form of bare chips between an inspection in the form of the sensor wafer 100 and a final inspection after the bare chips 10 are packaged into the solid-state image pickup apparatus.

The inspection in the form of bare chips is performed because dust and the like generated when each bare chip 10 is separated from the sensor wafer 100 by dicing or the like may adhere to the image area 11. If the bare chip 10 is packaged while dust and the like are adherent to the image area 11, a defect in the bare chip 10 is not found until a final inspection in the form of packages. This reduces manufacturing yield in addition to manufacturing efficiency.

A method including putting each bare chip into a socket in which a plurality of electronic components connectable to an inspection apparatus and actually used in an image pickup apparatus are mounted is conceivable as a method for the inspection in the form of bare chips. With the method, an inspection similar to the final inspection can be performed even in the form of bare chips, which allows a reduction in the number of components to be inspected and early detection of a failure. Accordingly, a reduction in a cost of a solid-state image pickup apparatus can be achieved.

When bare chips are to be transported at the time of the inspection in the form of bare chips, a socket into which the bare chips are put may be transported, and the small bare chips need not be directly transported. This not only improves transportability of bare chips but also prevents bare chips from being damaged at the time of transportation. The manufacturing yield thus increases.

Since a socket prevents a foreign substance from entering the bare chips 10 at the time of transportation, the manufacturing yield increases.

After the operator electrically connects the FPC 30 for inspection to the external connection terminals 21, the operator attaches a cover glass 15 to the front surface 10a of each bare chip 10 determined as a good chip in the inspection step to seal the image area 11, as shown in Fig. 3.

If a semiconductor device is to be formed to be smaller in the depth direction H than a general-purpose one after that, as described above, the operator performs a cutoff step of cutting off the external connection terminals 21 in the thickness direction A along line S1-S1, as shown in Fig. 3.

Finally, the operator performs a mounting step of electrically connecting the FPC 32 for mounting to the external connection terminals 22 by wire bonding using the wires 33, as shown in Fig. 4. After that, the operator packages the bare chip 10 with the external connection terminals 21 cut off to manufacture the solid-state image pickup apparatus.

On the other hand, if a general-purpose solid-state image pickup apparatus is to be manufactured, as described above, the operator electrically connects the FPC 30 for inspection to the external connection terminals 21 and uses the FPC 30 for inspection as an FPC for mounting without replacement. That is, in this case, the FPC 30 for inspection constitutes an FPC 40 which doubles as the FPC 32 for mounting, and the external connection terminals 21 are configured to double as the external connection terminals 22.

In that case, the above-described inspection step in the form of bare chips is unnecessary. This is because only the external connection terminals 21 are used for mounting FPCs on the bare chip 10, and an inspection to be performed using the external connection terminals 21 is the same as the inspection in the final step.

Accordingly, when a general-purpose solid-state image pickup apparatus is to be manufactured, the operator packages each bare chip 10 while the FPC 40 is electrically connected to the external connection terminals 21 by wire bonding using wires 41. With the operation, the operator manufactures the solid-state image pickup apparatus.

Consequently, if the FPC 40 is electrically connected to the external connection terminals 21, as shown in Fig. 5, the bare chip 10, including the FPC 40, has a length of H1 in the depth direction H. On the other hand, if the FPC 32 is electrically connected to the external connection terminals 22, the bare chip 10, including the FPC 32, has a length of H2 shorter by the cut-off external connection terminals 21 than H1 (H1 > H2) in the depth direction H. The solid-state image pickup apparatus can be made smaller in the depth direction H.

Note that in each of the FPCs 32 and 40 for mounting, for example, one surface of the FPC 32 or 40 is equipped with electronic components 161 such as a capacitor, a transistor, and a resistor, and lead wires 163 extending from a signal cable 334 (see Fig. 22) (to be described later) for transmitting and receiving an image pickup signal are connected to connection portions 162 which are provided at a surface opposite to the one surface with solder or the like. As described above, the wires 41 or 33 are electrically connected to the external connection terminals 21 or 22 of the bare chip 10 by wire bonding, and the connection portion is arranged at a rear of the bare chip 10 while the connection portions are covered with a sealing resin 160.

For the reason, in a solid-state image pickup apparatus 190 which is packaged by being connected to the FPC 32 or 40, the FPC 32 or 40 for mounting functions to transmit and receive an electrical signal such as an image pickup signal from a signal processing circuit (not shown) to and from the bare chip 10 via the signal cable 334 and the lead wires 163.

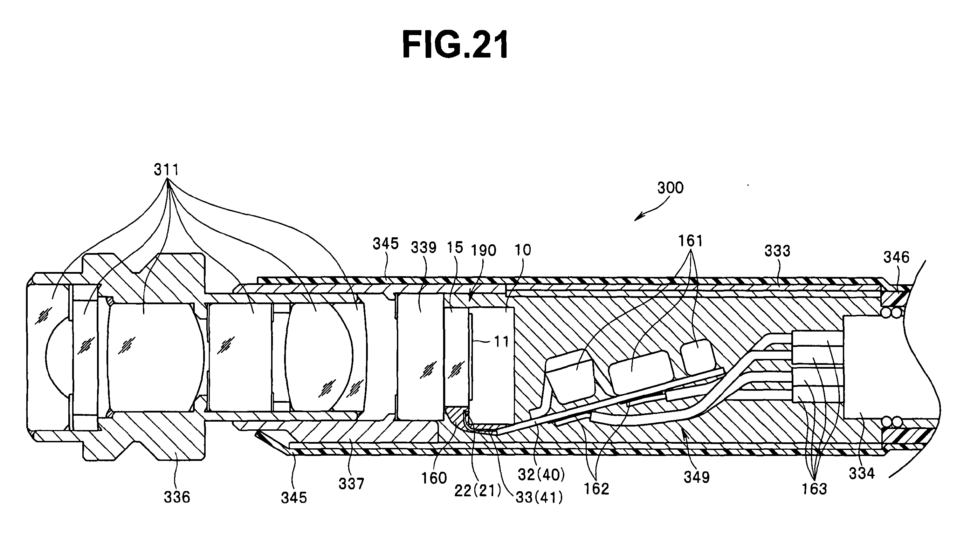

Note that the solid-state image pickup apparatus 190 with the above-described configuration is provided in an image pickup unit. Fig. 21 is a sectional view showing an example of an image pickup unit provided with the solid-state image pickup apparatus in Fig. 20.

As shown in Fig. 21, a main part of an image pickup unit 300 is composed of a group of objective lenses 311 including a plurality of objective lenses, a lens frame 336 which holds the group of objective lenses 311, the solid-state image pickup apparatus 190, a cover glass 339 which is attached to a part on a distal end side of the cover glass 15 of the solid-state image pickup apparatus 190 with UV adhesive or the like, an element frame 337 which holds the cover glass 339, a shielding material 333, the signal cable 334, heat-shrinkable tubing 345, protective tubing 346, and a thermoplastic resin 349.

A part on a rear end side in an insertion direction of an outer periphery of the lens frame 336 fits on a part on a distal end side of an inner periphery of the element frame 337 to be fixed, and a part on a distal end side of the shielding material 333 is fixed to a part on a rear end side of an outer periphery of the element frame 337.

In addition, a part on a distal end side of the heat-shrinkable tubing 345 covering the outer periphery of the element frame 337 and an outer periphery of the shielding material 333 is fixed to a part on the distal end side of the outer periphery of the element frame 337. A part on a rear end side of the heat-shrinkable tubing 345 is fixed to a part on a distal end side of an outer periphery of the protective tubing 346. Note that the protective tubing 346 protects the signal cable 334 by coating an outer periphery of the signal cable 334.

The solid-state image pickup apparatus 190 is disposed together with the thermoplastic resin 349 in an airtight space blockaded by the shielding material 333 and the heat-shrinkable tubing 345 at a rear of the group of objective lenses 311 in the insertion direction.

The solid-state image pickup apparatus 190 is fixed to the element frame 337 because of an outer periphery of the cover glass 339 fixed to the inner periphery of the element frame 337. Note that the image pickup unit, in which the solid-state image pickup apparatus 190 is provided, is not limited to the configuration in Fig. 21.

As described above, the present embodiment has described that the external connection terminals 22 for mounting are formed on the front surface 10a of each bare chip 10 and that the external connection terminals 21 for inspection doubling as ones for mounting are formed closer to the outer edge 10g of the bare chip 10 than the external connection terminals 22.

With the configuration, when a general-purpose solid-state image pickup apparatus is manufactured, the FPC 40 is electrically connected to the external connection terminals 21. On the other hand, when a solid-state image pickup apparatus smaller in the depth direction H than a general-purpose one is manufactured, the FPC 32 can be used while being electrically connected to the external connection terminals 22 after the external connection terminals 21 are cut off. This makes it possible to provide a solid-state image pickup apparatus with a configuration which allows each bare chip 10 to be formed to have either one of the two sizes without changing a size of the image sensor provided in the image area 11 and a method for manufacturing the solid-state image pickup apparatus.

Consequently, not only manufacturing costs and manufacturing control costs can be made lower than a conventional case where two types of bare chips are separately manufactured but also simplification of a manufacturing process can be achieved.

Even if a solid-state image pickup apparatus smaller in the depth direction H than a general-purpose one is to be manufactured, each bare chip 10 has the same size as a size of each general-purpose bare chip 10 of the general-purpose image pickup apparatus before the external connection terminals 21 are cut off. This not only improves the transportability of the bare chips 10 but also makes the bare chips 10 easier to handle.

When a solid-state image pickup apparatus smaller than a general-purpose one is manufactured, the FPC 30 for inspection can be electrically connected to the external connection terminals 21, and the bare chips 10 can be inspected in the form of bare chips, between the inspection in the form of the sensor wafer 100 and the final inspection after the bare chips 10 are packaged. Since the bare chips 10 can be checked for defects before the final inspection, the manufacturing yield can be increased.

Note that although the present embodiment has described that two groups of external connection terminals are formed on the front surface 10a of each bare chip 10, the present invention is not limited to this. Of course, two or more groups of external connection terminals may be formed.

### (Second Embodiment)

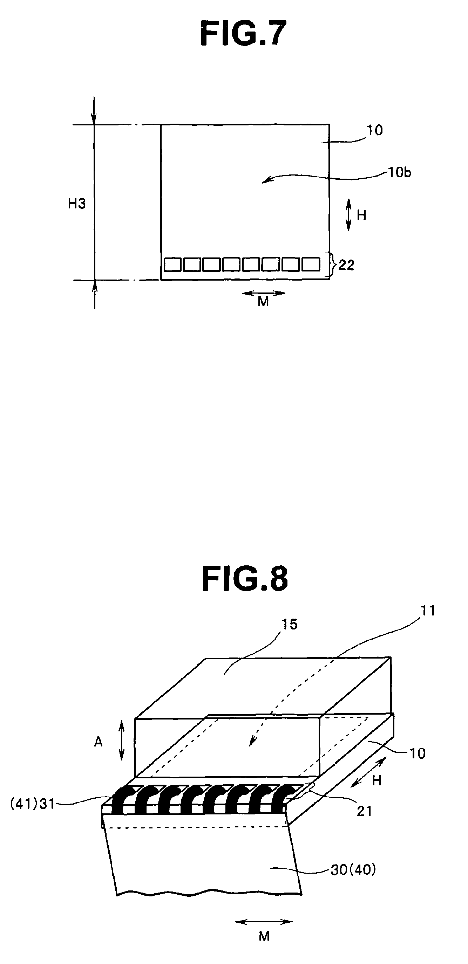

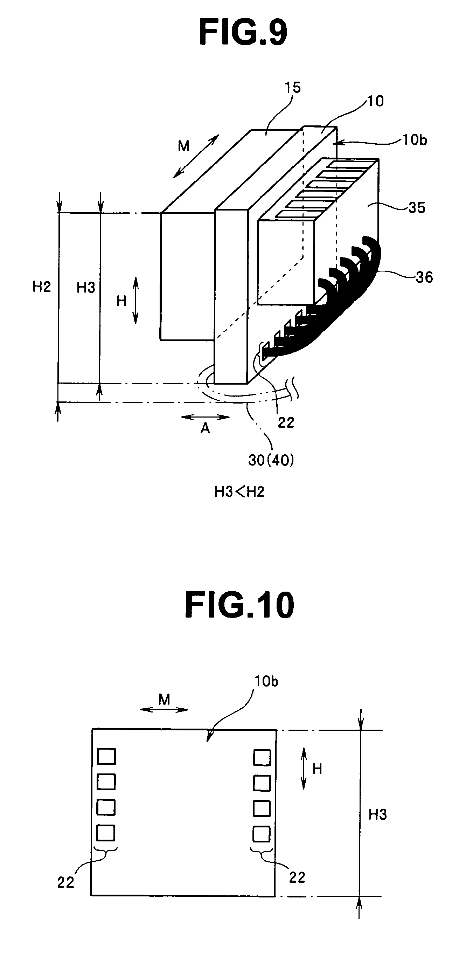

Fig. 6 is a plan view showing a bare chip used in a solid-state image pickup apparatus according to the present embodiment as seen from a first surface side. Fig. 7 is a rear view of the bare chip in Fig. 6 as seen from a second surface side. Fig. 8 is a partial perspective view showing a state in which a cover glass is attached to cover an image area in Fig. 6 and a substrate for inspection is mounted on a first group of terminals. Fig. 9 is a perspective view showing a state in which a substrate for mounting is mounted on a second group of terminals in Fig. 7.

A configuration of the solid-state image pickup apparatus according to the second embodiment is different from the solid-state image pickup apparatus according to the first embodiment shown in Figs. 1 to 5 in that external connection terminals to which the substrate for mounting is electrically connected are formed at a back surface of each bare chip.

Accordingly, only the difference between the embodiments will be described. The same components as the components in the first embodiment are denoted by the same reference numerals, and a description of the components will be omitted.

As shown in Fig. 6, only external connection terminals 21 are formed along a width direction M in a region outside an image area 11 at a front surface 10a of a bare chip 10 of the solid-state image pickup apparatus according to the present embodiment. As shown in Fig. 7, external connection terminals 22 are formed along the width direction M at a back surface 10b which is a second surface opposite to the front surface 10a. Note that since functions of the external connection terminals 21 and 22 are similar to the functions in the first embodiment, a description of the functions will be omitted.

Effects of the present embodiment with the above-described configuration will be described.

When the solid-state image pickup apparatus is to be manufactured, an operator first cuts the plurality of bare chips 10 from a sensor wafer 100 including the plurality of bare chips 10, each having the image area 11 and the external connection terminals 21 and 22, as shown in Fig. 1, by, e.g., dicing, as shown in Figs. 6 and 7.

The operator electrically connects an FPC 30 for inspection to the external connection terminals 21 formed on the front surface 10a of each bare chip 10 by wire bonding using wires 31 and inputs a signal from an external apparatus to the bare chip 10 via the FPC 30, as shown in Fig. 8. With the operation, the operator performs the inspection step of the driving inspection of the bare chip 10.

After the operator electrically connects the FPC 30 to the external connection terminals 21, the operator attaches a cover glass 15 to the front surface 10a of each bare chip 10 determined as a good chip in the inspection step to seal the image area 11, as shown in Fig. 8.

If a semiconductor device is to be formed to be smaller in a depth direction H than a general-purpose one after that, as described above, the operator cuts off the wires 31. After that, the operator performs a mounting step of attaching a multilayer substrate 35 to the back surface 10b of the bare chip 10 and electrically connecting the multilayer substrate 35 to the external connection terminals 22 formed on the back surface 10b by wire bonding using wires 36, as shown in Fig. 9. After that, the operator packages the bare chip 10 to manufacture the solid-state image pickup apparatus.

On the other hand, if a general-purpose solid-state image pickup apparatus is to be manufactured, as described above, the operator electrically connects the FPC 30 for inspection to the external connection terminals 21 and uses the FPC 30 for inspection as an FPC for mounting without replacement. That is, in this case, the FPC 30 for inspection constitutes an FPC 40 which doubles as an FPC 32 for mounting, and the external connection terminals 21 are configured to double as the external connection terminals 22.

In the present embodiment as well, the above-described inspection step in the form of bare chips is unnecessary in that case. This is because only the external connection terminals 21 are used for mounting FPCs on the bare chip 10, and an inspection to be performed using the external connection terminals 21 is the same as an inspection in the final step.

Note that if a general-purpose solid-state image pickup apparatus is to be manufactured, the operator may electrically connect the FPC 32 for mounting to the external connection terminals 21 at the front surface 10a after the operator electrically connects the FPC 30 for inspection to the external connection terminals 22 at the back surface 10b and performs an inspection in the form of bare chips.

Accordingly, when a general-purpose solid-state image pickup apparatus is to be manufactured, the operator packages each bare chip 10 while the FPC 40 is electrically connected to the external connection terminals 21 by wire bonding using wires 41. With the operation, the operator manufactures the solid-state image pickup apparatus.

Consequently, if the FPC 40 is electrically connected to the external connection terminals 21 at the front surface 10a, the bare chip 10, including the FPC 40, has a length of H2 in the depth direction H. On the other hand, if the multilayer substrate 35 is electrically connected to the external connection terminals 22 at the back surface 10b, the bare chip 10 has a length of H3 shorter than H2 (H2 > H3) in the depth direction H. The solid-state image pickup apparatus can be made smaller in the depth direction H.

If the FPC 40 is electrically connected to the external connection terminals 21 at the front surface 10a, the bare chip 10 can be formed to be shorter on the whole, i.e., smaller in a thickness direction A than the case where the multilayer substrate 35 is electrically connected to the external connection terminals 22 at the back surface 10b. This is especially effective when a solid-state image pickup apparatus is to be formed to be thin in the thickness direction A, e.g., so as to be incorporated in a mobile phone.

Accordingly, when a small solid-state image pickup apparatus is to be manufactured, how a substrate is connected to the external connection terminals 21 and 22 may be selected depending on whether a size reduction in the thickness direction A is desired or a size reduction in the depth direction H is desired.

As described above, the present embodiment has described that the external connection terminals 21 are formed on the front surface 10a of each bare chip 10 and the external connection terminals 22 are formed on the back surface 10b of the bare chip 10.

With the configuration, a process of attaching the multilayer substrate 35 to the back surface 10b of the bare chip 10 and electrically connecting the multilayer substrate 35 to the external connection terminals 22 by wire bonding using the wires 36 can make the solid-state image pickup apparatus smaller in the depth direction H than a process of electrically connecting the FPC 40 to the external connection terminals 21 by wire bonding using the wires 41. The process of electrically connecting the FPC 40 to the external connection terminals 21 can make the solid-state image pickup apparatus smaller in the thickness direction A than the process of electrically connecting the multilayer substrate 35 to the external connection terminals 22. That is, which direction a size of the solid-state image pickup apparatus is reduced can be selected by selecting how the FPC is connected to the external connection terminals 21 and 22.

As can been seen from the above, a solid-state image pickup apparatus with a configuration which allows each bare chip 10 to be formed to have either one of the two sizes without cutting off a part of the bare chip 10 and changing a size of an image sensor and a method for manufacturing the solid-state image pickup apparatus can be provided. Note that other advantages are similar to those of the first embodiment above.

A case where one group of external connection terminals 21 are formed on the front surface 10a of each bare chip 10 has been described as an example in the present embodiment. However, the present invention is not limited to this, and two or more groups of external connection terminals 21 may be formed. In addition, two or more groups of external connection terminals 22 may be formed on the back surface 10b of the bare chip 10.

### (Third Embodiment)

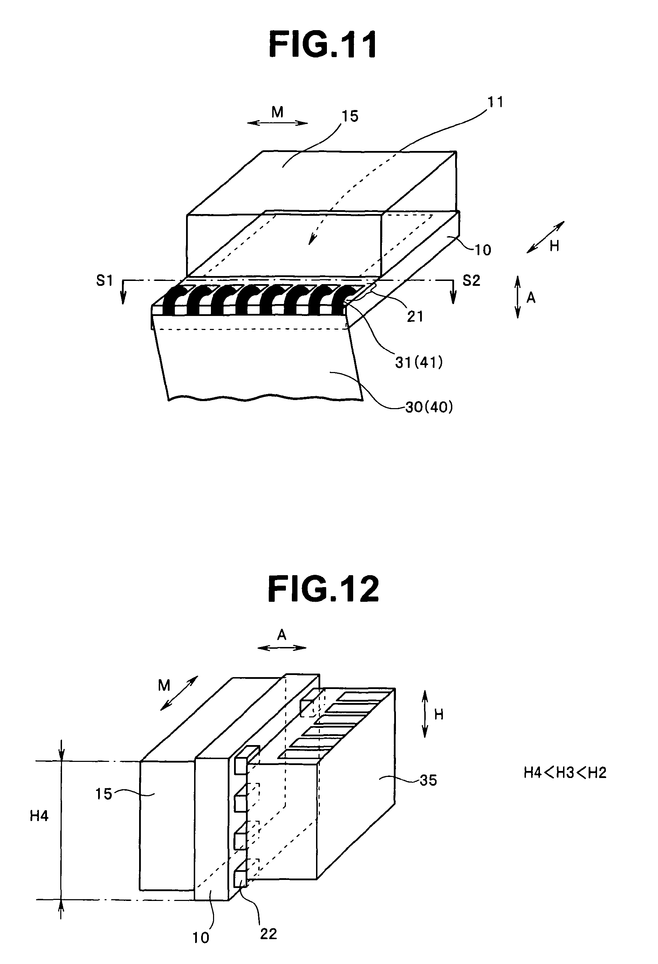

Fig. 10 is a rear view of a bare chip used in a semiconductor device according to the present embodiment as seen from a back surface side. Fig. 11 is a partial perspective view showing a state in which a cover glass is attached to cover an image area on a front surface side in Fig. 10 and a substrate for inspection is mounted on a first group of terminals. Fig. 12 is a perspective view showing a state in which a substrate for mounting is mounted on a second group of terminals in Fig. 10.

A configuration of a solid-state image pickup apparatus according to the third embodiment is different from the solid-state image pickup apparatus according to the second embodiment shown in Figs. 6 to 9 in that external connection terminals to which a substrate for mounting formed on a back surface of a bare chip is electrically connected are formed, in a plan view, at positions that do not overlap with external connection terminals to which a substrate for inspection is electrically connected.

Accordingly, only the difference between the embodiments will be described. The same components as the components in the second embodiment are denoted by the same reference numerals, and a description of the components will be omitted.

As shown in Fig. 6 described above, only external connection terminals 21 are formed along a width direction M in a region outside an image area 11 at a front surface 10a of a bare chip 10 of a solid-state image pickup apparatus according to the present embodiment. As shown in Fig. 10, external connection terminals 22 are formed along a depth direction H in a region which does not overlap with the external connection terminals 21 in a plan view from a thickness direction A of a back surface 10b or, more specifically, at positions on two end sides in the width direction M of the bare chip 10, which allow only the external connection terminals 21 to be cut off. Note that since functions of the external connection terminals 21 and 22 are the same as the functions in the second embodiment, a description of the functions will be omitted.

Effects of the present embodiment with the above-described configuration will be described.

When solid-state image pickup apparatuses are to be manufactured, an operator first cuts the plurality of bare chips 10 from a sensor wafer 100 including the plurality of bare chips 10, each having the image area 11 and the external connection terminals 21 and 22, as shown in Fig. 1, by, e.g., dicing, as shown in Fig. 10.

The operator electrically connects an FPC 30 for inspection to the external connection terminals 21 formed on the front surface 10a of each bare chip 10 by wire bonding using wires 31 and inputs a signal from an external apparatus to the bare chip 10 via the FPC 30, as shown in Fig. 11. With the operation, the operator performs the inspection step of the driving inspection of the bare chip 10.

After the operator electrically connects the FPC 30 to the external connection terminals 21, the operator attaches a cover glass 15 to the front surface 10a of each bare chip 10 determined as a good chip in the inspection step to seal the image area 11, as shown in Fig. 11.

If a semiconductor device is to be formed to be smaller in the depth direction H than a general-purpose one after that, as described above, the operator performs a mounting step of directly mounting a multilayer substrate 35 on the external connection terminals 22 formed on the back surface 10b of the bare chip 10 by using a BGA or the like, as shown in Fig. 12. The operator cuts off the external connection terminals 21 of the bare chip 10 in the thickness direction A along line S2-S2, as shown in Fig. 11. After that, the operator packages the bare chip 10 to manufacture the solid-state image pickup apparatus.

On the other hand, if a general-purpose solid-state image pickup apparatus is to be manufactured, as described above, the operator electrically connects the FPC 30 for inspection to the external connection terminals 21 and uses the FPC 30 for inspection as an FPC for mounting without replacement. That is, in the case, the FPC 30 for inspection constitutes an FPC 40 which doubles as an FPC 32 for mounting, and the external connection terminals 21 are configured to double as the external connection terminals 22.

In the present embodiment as well, the above-described inspection step in the form of bare chips is unnecessary in this case. This is because only the external connection terminals 21 are used for mounting FPCs on the bare chip 10, and an inspection to be performed using the external connection terminals 21 is the same as an inspection in the final step.

Accordingly, when a general-purpose solid-state image pickup apparatus is to be manufactured, the operator packages each bare chip 10 while the FPC 40 is electrically connected to the external connection terminals 21 by wire bonding using wires 41. With the operation, the operator manufactures the solid-state image pickup apparatus.

Consequently, if the FPC 40 is electrically connected to the external connection terminals 21 at the front surface 10a, as shown in Fig. 11, the bare chip 10, including the FPC 40, has a length of H2 in the depth direction H, as shown in Fig. 9. On the other hand, if the multilayer substrate 35 is electrically connected to the external connection terminals 22 at the back surface 10b, the bare chip 10 has a length of H4 shorter by the cut-off external connection terminals 21 than H2 and H3 (H2 > H3 > H4) in the depth direction H. The solid-state image pickup apparatus can be made smaller in the depth direction H than that of the second embodiment.

If the FPC 40 is electrically connected to the external connection terminals 21 at the front surface 10a, the bare chip 10 can be formed to be shorter on the whole, i.e., smaller in the thickness direction A than the case where the multilayer substrate 35 is electrically connected to the external connection terminals 22 at the back surface 10b. This is especially effective when a solid-state image pickup apparatus is to be formed to be thin in the thickness direction A, e.g., so as to be incorporated in a mobile phone.

Accordingly, when a small solid-state image pickup apparatus is to be manufactured, how a substrate is connected to the external connection terminals 21 and 22 may be selected depending on whether a size reduction in the thickness direction A is desired or a size reduction in the depth direction H is desired.

As described above, the present embodiment has described that the external connection terminals 21 are formed on the front surface 10a of each bare chip 10 and the external connection terminals 22 are formed in a region which does not overlap with the external connection terminals 21 in a plan view from the thickness direction A, on the back surface 10b of the bare chip 10.

With the configuration, a process of directly mounting the external connection terminals 22 on the multilayer substrate 35 and cutting off the external connection terminals 21 after an inspection can make the solid-state image pickup apparatus smaller in the depth direction H by the external connection terminals 21 cut off in the depth direction H than a process of electrically connecting the FPC 40 to the external connection terminals 21 by wire bonding using the wires 41, i.e., smaller than the solid-state image pickup apparatus the second embodiment. Additionally, the process of electrically connecting the FPC 40 to the external connection terminals 21 can make the solid-state image pickup apparatus smaller in the thickness direction A. That is, which direction a size of the solid-state image pickup apparatus is reduced can be selected by selecting how the substrate is connected to the external connection terminals 21 and 22.

As can been seen from the above, a solid-state image pickup apparatus with a configuration which allows the bare chip 10 to be formed to have either one of the two sizes without changing a size of an image sensor and a method for manufacturing the solid-state image pickup apparatus can be provided. Note that other advantages are the same as those of the second embodiment above.

A case where two rows of the external connection terminals 22 are formed on the back surface 10b of each bare chip 10 has been described as an example in the present embodiment. However, the present invention is not limited to this, and two or more rows of the external connection terminals 22 may be formed.

### (Fourth Embodiment)

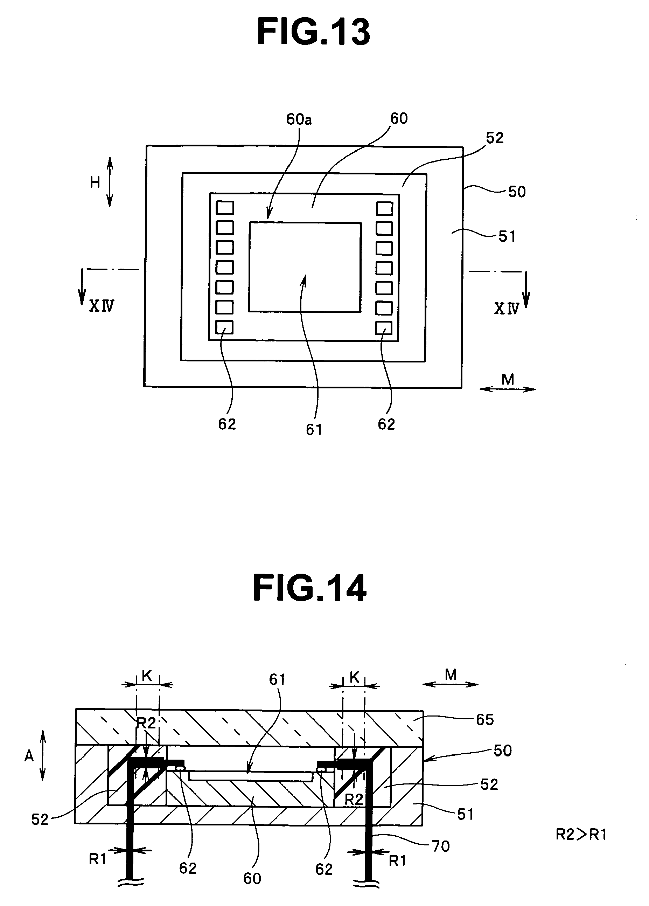

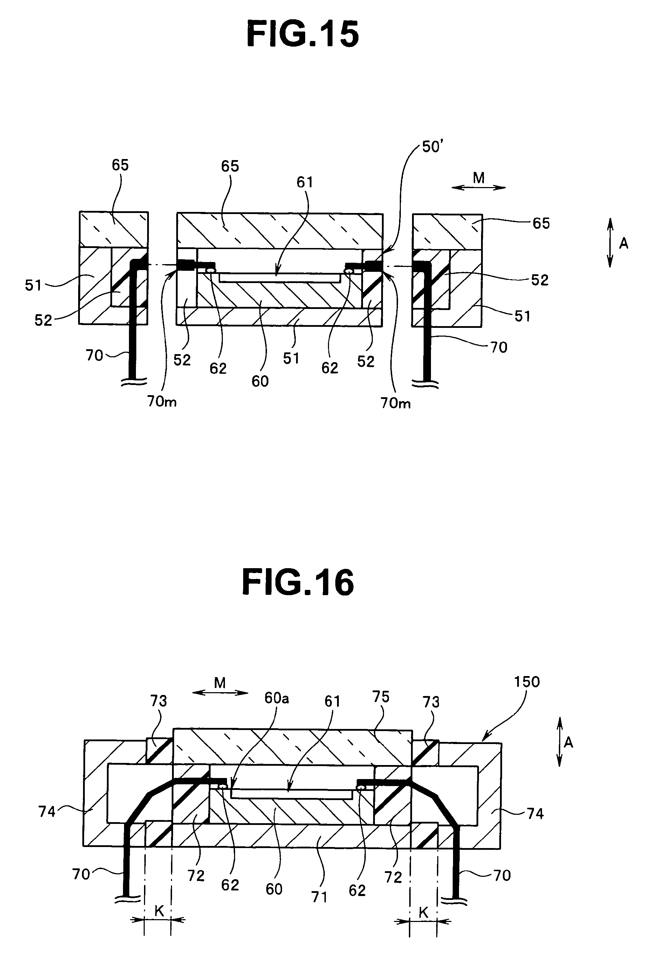

Fig. 13 is a plan view showing a semiconductor package in a solid-state image pickup apparatus, according to the present embodiment. Fig. 14 is a sectional view of the semiconductor package taken along line XIV-XIV in Fig. 13. Fig. 15 is an exploded perspective view showing a state in which parts of the semiconductor package in Fig. 14 are cut off at cutoff regions formed in the semiconductor package.

A configuration of the solid-state image pickup apparatus according to the fourth embodiment is different from the solid-state image pickup apparatuses according to the first to third embodiments shown in Figs. 1 to 12 in that the configuration allows a semiconductor package including a bare chip to be formed to have either one of two sizes without changing a size of an image sensor.

As shown in Figs. 13 and 14, in a semiconductor package (hereinafter simply referred to as a package) 50 in the solid-state image pickup apparatus according to the present embodiment, a bare chip 60 serving as a semiconductor chip is stored in a space sealed with a cover glass 65 inside a storage case 51 serving as a storage portion and made of, e.g., ceramic.

External connection terminals 62 are formed along a depth direction H at two ends in a width direction M outside an image area 61 which is an active region at a front surface 60a of a bare chip 60, as shown in Fig. 13.

A wire 70 extending from a substrate which drives the bare chip 60 by inputting a signal from an external apparatus to the bare chip 60 is electrically connected to each external connection terminal 62 by wire bonding and extends outside the package 50, as shown in Fig. 14.

In the storage case 51, a region around the bare chip 60 is filled with a sealing material 52 made of, e.g., resin, as shown in Figs. 13 and 14. Accordingly, a part of the wire 70 extending from each external connection terminal 62, which is inside the storage case 51, is covered by the sealing material 52.

As shown in Fig. 14, cutoff regions K for cutting off regions which do not include the bare chip 60 in a plan view along a thickness direction A of the package 50 are provided in the package 50. More specifically, each cutoff region K is provided in the region filled with the sealing material 52 of the package 50 in a plan view.

Each wire 70 is formed such that a wire diameter R2 of a part in the cutoff region K of each wire 70 is larger than a wire diameter R1 of another wire part (R2 > R1).

Effects of the present embodiment with the above-described configuration will be described.

When the solid-state image pickup apparatus is to be manufactured, an operator first cuts the plurality of bare chips 60 from a sensor wafer 100 including the plurality of bare chips 60, each having the image area 61 and the external connection terminals 62, as shown in Fig. 1, by, e.g., dicing.

As shown in Figs. 13 and 14, after the operator stores each bare chip 60 in the storage case 51, the operator electrically connects the wire 70 to each external connection terminal 62 by wire bonding. The operator fills a region around the bare chip 60 in the storage case 51 with the sealing material 52. After that, the operator attaches the cover glass 65 to the storage case 51 so as to seal the sealing material 52 and the bare chip 60 in the storage case 51. As a result, the above-described package 50 for a general-purpose solid-state image pickup apparatus is manufactured.

If a solid-state image pickup apparatus smaller than a general-purpose solid-state image pickup apparatus is to be manufactured after that, the operator performs a cutoff step of cutting off parts of the package 50 at the cutoff regions K formed in the package 50 along the thickness direction A with a diamond cutter or the like, as shown in Fig. 15. More specifically, the operator performs the cutoff step so as to cut off the large-diameter part having the wire diameter R2 of each wire 70.

Consequently, a package 50' shown in Fig. 15, which is smaller in the width direction M, than the general-purpose package shown in Fig. 14 is manufactured.

Finally, after the cutoff, the operator electrically connects an FPC, a cable, or the like connectable to an external apparatus to an exposed part 70m of the wire 70 which is exposed from a side in the width direction M of the package 50'. In this manner, the operator uses the package 50'.

As described above, the present embodiment has described that the cutoff regions K are formed in the package 50, which is formed by storing the bare chip 60 and the sealing material 52 in the storage case 51 sealed with the cover glass 65. More specifically, the present embodiment has described that each cutoff region K is formed in a region overlapping with the large-diameter parts of the corresponding wires 70 in a plan view.

With the configuration, if a solid-state image pickup apparatus smaller than a general-purpose solid-state image pickup apparatus is desired to be manufactured, parts of the package 50 may be cut off at the cutoff regions K along the thickness direction A so as to cut off the large-diameter parts of the wires 70. Either one of the two types of solid-state image pickup apparatus different in a size in the width direction M can be easily manufactured even from a package.

Accordingly, a solid-state image pickup apparatus with a configuration which allows a package to be formed to have either one of the two sizes without changing a size of an image sensor and a method for manufacturing the solid-state image pickup apparatus can be provided.

Even if a small solid-state image pickup apparatus is to be manufactured, a package before cutoff at the cutoff regions K has the same size as a size of a general-purpose package and is easy to handle.

After the cutoff, since the exposed part 70m of each wire 70 is a large-diameter part, end face processing on the exposed part or, more specifically, a process of connecting the FPC or the cable to the exposed part is easier.

### (Fifth Embodiment)

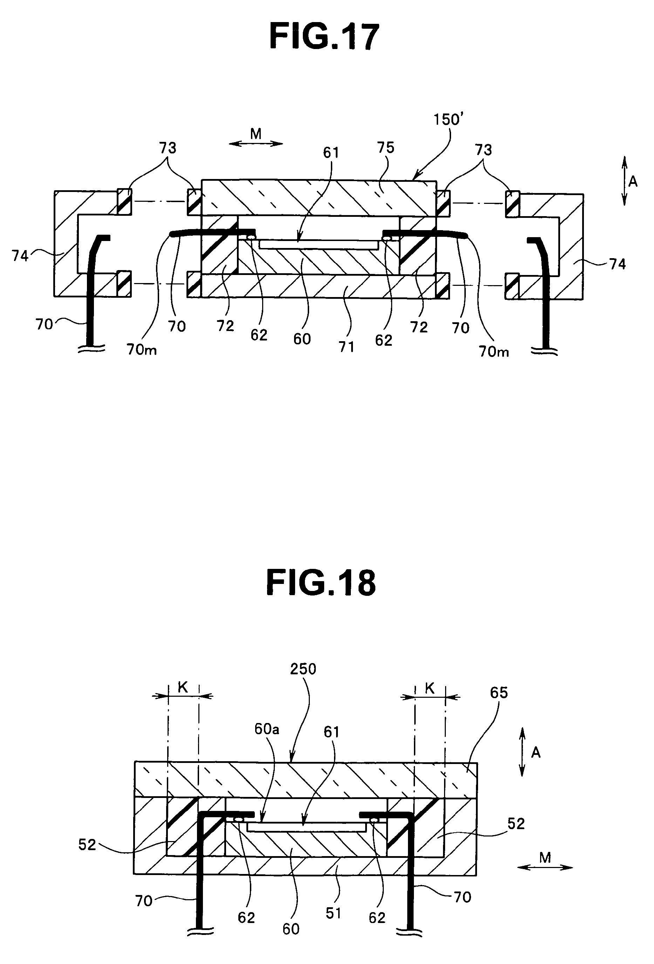

Fig. 16 is a sectional view showing a semiconductor package in a solid-state image pickup apparatus, according to the present embodiment. Fig. 17 is an exploded perspective view showing a state in which parts of the semiconductor package in Fig. 16 are cut off at cutoff regions formed in the semiconductor package.

A configuration of the solid-state image pickup apparatus according to the fifth embodiment is different from the solid-state image pickup apparatus according to the fourth embodiment described above shown in Figs. 13 to 15 in a configuration of a package and a position of a cutoff region.

Accordingly, only the difference between the embodiments will be described. The same components as the components in the fourth embodiment are denoted by the same reference numerals, and a description of the components will be omitted.

As shown in Fig. 16, in a semiconductor package (hereinafter simply referred to as a package) 150 in the solid-state image pickup apparatus according to the present embodiment, a bare chip 60 serving as a semiconductor chip is placed in a storage case 71 serving as a storage portion and made of, e.g., ceramic. On the storage case 71, a region around the bare chip 60 is filled with a first sealing material 72 made of, e.g., resin. A cover glass 75 is attached to an upper surface in a thickness direction A of the first sealing material 72 so as to overlap with an image area 61 in a plan view.

A frame 74 is attached via a second sealing material 73 made of, e.g., resin to each side in a width direction M of a structure which is composed of the storage case 71, the bare chip 60, the first sealing material 72, and the cover glass 75 with the above-described configuration. The package 150 includes the above-described components.

In the present embodiment as well, external connection terminals 62 (not shown) are formed along a depth direction H at two ends in the width direction M outside the image area 61 that is an active region at a front surface 60a of the bare chip 60.

A wire 70 extending from a substrate which drives the bare chip 60 by inputting a signal from an external apparatus to the bare chip 60 is electrically connected to each external connection terminal 62 by wire bonding and extends outside the package 150.

As shown in Fig. 16, cutoff regions K for cutting off regions which do not include the bare chip 60 in a plan view along the thickness direction A of the package 150 are provided in the package 150. More specifically, each cutoff region K is provided at the second sealing material 73.

Effects of the present embodiment with the above-described configuration will be described.

When the solid-state image pickup apparatus is to be manufactured, an operator first cuts the plurality of bare chips 60 from a sensor wafer 100 including the plurality of bare chips 60, each having the image area 61 and the external connection terminals 62, as shown in Fig. 1, by, e.g., dicing.

As shown in Fig. 16, after the operator places each bare chip 60 in the storage case 71, the operator electrically connects the wire 70 to each external connection terminals 62 by wire bonding. The operator fills a space around the bare chip 60 on the storage case 71 with the first sealing material 72. After that, the operator attaches the cover glass 75 to the first sealing material 72 so as to seal the bare chip 60.

Finally, the operator attaches the frame 74 via the second sealing material 73 to each side in the width direction M of the structure with the cover glass 75 attached. In the manner, the above-described package 150 for a general-purpose solid-state image pickup apparatus is manufactured.

If a solid-state image pickup apparatus smaller than a general-purpose solid-state image pickup apparatus is to be manufactured after that, the operator performs a cutoff step of cutting off parts of the package 150 at the cutoff regions K formed in the package 150 along the thickness direction A in a manner as shown in Fig. 17. More specifically, the operator performs the cutoff step so as to cut off parts at the second sealing materials 73.

Consequently, a package 150' shown in Fig. 17 which is smaller in the width direction M than the general-purpose package 150 shown in Fig. 16 is manufactured.

Finally, after the cutoff, the operator electrically connects an FPC, a cable, or the like connectable to an external apparatus to an exposed part 70m of a wire 70 which is exposed from a side in the width direction M of the package 150'. In this manner, the operator uses the package 50'.

As described above, the present embodiment has described that the cutoff regions K are provided at the second sealing materials 73 of the package 150.

With the configuration, if a solid-state image pickup apparatus smaller than a general-purpose solid-state image pickup apparatus is desired to be manufactured, parts of the package 150 may be cut off at the cutoff regions K along the thickness direction A so as to cut off parts at the second sealing materials 73. Either one of the two types of solid-state image pickup apparatus different in a size in the width direction M can be easily manufactured even from a package.

Accordingly, a solid-state image pickup apparatus with a configuration which allows a package to be formed to have either one of the two sizes without changing a size of an image sensor and a method for manufacturing the solid-state image pickup apparatus can be provided.

Note that other advantages are the same as those of the fourth embodiment above.

### (Sixth Embodiment)

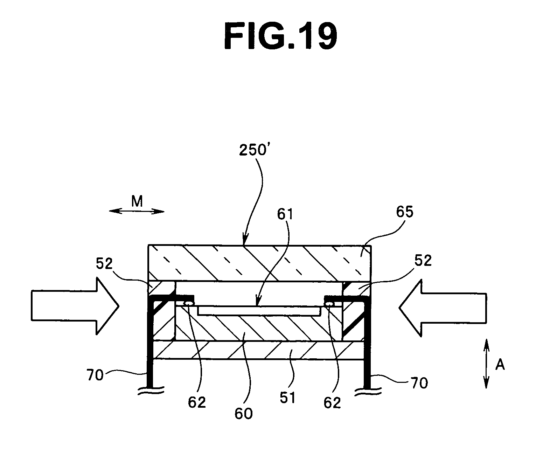

Fig. 18 is a sectional view showing a semiconductor package in a solid-state image pickup apparatus, according to the present embodiment. Fig. 19 is an exploded perspective view showing a state in which parts of the semiconductor package in Fig. 18 are cut off at cutoff regions formed in the semiconductor package.

A configuration of the solid-state image pickup apparatus according to the sixth embodiment is different from the solid-state image pickup apparatus according to the fourth embodiment described above shown in Figs. 13 to 15 only in that a cutoff region is formed outside a wire extending part along a thickness direction.

Accordingly, only the difference between the embodiments will be described. The same components as the components in the fourth embodiment are denoted by the same reference numerals, and a description of the components will be omitted.

As shown in Fig. 18, in a semiconductor package (hereinafter simply referred to as a package) 250 in the solid-state image pickup apparatus according to the present embodiment, a bare chip 60 serving as a semiconductor chip is stored in a space sealed with a cover glass 65 inside a storage case 51 serving as a storage portion and made of, e.g., ceramic.

External connection terminals 62 (not shown) are formed along a depth direction H at two ends in a width direction M outside an image area 61 that is an active region, on a front surface 60a of the bare chip 60.

A wire 70 extending from a substrate which drives the bare chip 60 by inputting a signal from an external apparatus to the bare chip 60 is electrically connected to each external connection terminal 62 by wire bonding. After the wire 70 extends outward from each external connection terminal 62 in the width direction M, the wire 70 is substantially perpendicularly bent so as to extend along a thickness direction A and extends outside the package 250, as shown in Fig. 18.

In the storage case 251, a region around the bare chip 60 is filled with a sealing material 52 made of, e.g., resin, as shown in Figs. 18 and 19.

As shown in Fig. 18, cutoff regions K for cutting off regions which do not include the bare chip 60 in a plan view along the thickness direction A of the package 250 are provided in the package 250. More specifically, each cutoff region K is provided outside the wire 70 extending along the thickness direction A in the region filled with the sealing material 52 of the package 250, in a plan view.

Effects of the present embodiment with the above-described configuration will be described.

When the solid-state image pickup apparatus is to be manufactured, an operator first cuts the plurality of bare chips 60 from a sensor wafer 100 including the plurality of bare chips 60, each having the image area 61 and the external connection terminals 62, as shown in Fig. 1, by, e.g., dicing.

As shown in Fig. 18, after the operator stores each bare chip 60 in the storage case 51, the operator electrically connects the wire 70 to each external connection terminal 62 by wire bonding. After the operator extends the wire 70 from each external connection terminal 62 in the width direction M, the operator substantially perpendicularly bends the wire 70 so as to extend along the thickness direction A and extends the wire 70 outside the package 250.

The operator fills a region around the bare chip 60 in the storage case 51 with the sealing material 52. After that, the operator attaches the cover glass 65 to the storage case 51 so as to seal the sealing material 52 and the bare chip 60 in the storage case 51. As a result, the above-described package 250 for a general-purpose solid-state image pickup apparatus is manufactured.

If a solid-state image pickup apparatus smaller than a general-purpose solid-state image pickup apparatus is to be manufactured after that, the operator performs a cutoff step of grinding away or cutting off the cutoff regions K formed in the package 250 along the thickness direction A in a manner as shown in Fig. 19. More specifically, the operator grinds or cuts the package 250 from an outward part in the width direction M to the wires 70 outside parts along the thickness direction A of the wires 70 to manufacture a package 250' shown in Fig. 19, which is smaller in the width direction M, than the general-purpose package 250 shown in Fig. 18.

As described above, the present embodiment has described that the cutoff regions K are formed in the package 250, which is formed by storing the bare chip 60 and the sealing material 52 in the storage case 51 sealed with the cover glass 65. More specifically, the present embodiment has described that each cutoff region K is formed outside the parts along the thickness direction A of the corresponding wires 70.

With the configuration, if a solid-state image pickup apparatus smaller than a general-purpose solid-state image pickup apparatus is desired to be manufactured, the package 250 may be cut at the cutoff regions K along the thickness direction A or the package 250 may be ground from the outward parts in the width direction M to the wires 70. Either one of the two types of solid-state image pickup apparatus different in a size in the width direction M can be easily manufactured even from a package.

Accordingly, a solid-state image pickup apparatus with a configuration which allows a package to be formed to have either one of the two sizes without changing a size of an image sensor and a method for manufacturing the solid-state image pickup apparatus can be provided. Note that other advantages are the same as those of the fourth embodiment above.

A solid-state image pickup apparatus has been described as an example of the semiconductor device in each of the first to the sixth embodiments described above. However, it will be appreciated that same advantages as those of the present embodiments can also be achieved by applying the present embodiments to other semiconductor devices.

The present application claims priority on the basis of Japanese Patent Application No. 2009-99206 filed in Japan on April 15, 2009, the entire contents of which are incorporated in the specification, claims, and drawings of the present application.

## Claims

1. A semiconductor device comprising a semiconductor chip,
wherein at least two or more groups of external connection terminals to which a substrate that drives the semiconductor chip by inputting a signal from an external apparatus to the semiconductor chip is electrically connected are formed in a region outside an active region of the semiconductor chip.

2. The semiconductor device according to claim 1, wherein among the at least two or more groups of external connection terminals, at least one group of terminals constitutes a first group of terminals on which a substrate for inspection used to inspect the semiconductor chip is mounted, and another group of terminals different from the first group of terminals constitutes a second group of terminals on which a substrate for mounting used to mount the semiconductor chip is mounted.

3. The semiconductor device according to claim 1 or 2, wherein the first group of terminals and the second group of terminals are formed on a first surface of the semiconductor chip, and
on the first surface, the first group of terminals is formed at positions which allow only the first group of terminals to be cut off in a part closer to an outer edge of the semiconductor chip than the second group of terminals.

4. The semiconductor device according to claim 1 or 2, wherein the first group of terminals is formed on the first surface of the semiconductor chip, and
the second group of terminals is formed at a second surface opposite to the first surface of the semiconductor chip.

5. The semiconductor device according to claim 4, wherein on the first surface, the first group of terminals is formed at positions which allow only the first group of terminals to be cut off in a region which does not overlap with the second group of terminals in a plan view.

6. The semiconductor device according to any one of claims 2 to 5, wherein the first group of terminals doubles as the second group of terminals, and the substrate for inspection doubles as the substrate for mounting, and
the substrate for inspection doubling as the substrate for mounting is electrically connected to the first group of terminals.

7. A semiconductor device comprising a semiconductor package in which a semiconductor chip is stored in a storage portion,
wherein a cutoff region for cutting off a region which does not include the semiconductor chip along a thickness direction of the semiconductor package is provided in the semiconductor package.

8. The semiconductor device according to claim 7, wherein a substrate which drives the semiconductor chip by inputting a signal from an external apparatus to the semiconductor chip is electrically connected to an external connection terminal of the semiconductor chip by wire bonding.

9. The semiconductor device according to claim 8, wherein the semiconductor package is configured such that a region around the semiconductor chip is filled with a sealing material in the storage portion sealed with a cover glass, and
the cutoff region is provided in the region filled with the sealing material in a plan view, and a wire used for the wire bonding is formed to be thicker in the cutoff region than at another position.

10. The semiconductor device according to claim 8, wherein the semiconductor package is configured such that a frame is attached via a second sealing material to a side along a thickness direction of a structure in which a region around the semiconductor chip placed in the storage portion is filled with a first sealing material, and a cover glass is attached to the first sealing material to overlap with the semiconductor chip in a plan view, and
the cutoff region is provided at the second sealing material.

11. The semiconductor device according to claim 8, wherein the semiconductor package is configured such that a region around the semiconductor chip is filled with a sealing material in the storage portion sealed with a cover glass, and a wire used for the wire bonding is formed to extend from the external connection terminal in a width direction of the semiconductor package and be substantially perpendicularly bent so as to extend along the thickness direction of the semiconductor package, and
the cutoff region is provided outside the wire in the width direction in a plan view at the sealing material.

12. A method for manufacturing a semiconductor device including a semiconductor chip, comprising:
an inspection step of electrically connecting a substrate for inspection to a first group of terminals constituted by at least one group of terminals among at least two or more groups of external connection terminals formed in a region outside an active region of the semiconductor chip and performing a driving inspection of the semiconductor chip by inputting a signal from an external apparatus to the semiconductor chip; and
a mounting step of electrically connecting a substrate for mounting to a second group of terminals different from the first group of terminals among the external connection terminals after the driving inspection ends.

13. The method for manufacturing a semiconductor device according to claim 12, comprising a cutoff step of cutting off the first group of terminals from the semiconductor chip after the inspection step and before the mounting step.

14. A method for manufacturing a semiconductor device including a semiconductor chip, comprising a step of electrically connecting a substrate for inspection which doubles as a substrate for mounting to a first group of terminals which doubles as a second group of terminals and is constituted by at least one group of terminals among at least two or more groups of external connection terminals formed in a region outside an active region of the semiconductor chip.

15. A method for manufacturing a semiconductor device including a semiconductor package in which a semiconductor chip is stored in a storage portion, comprising:
performing a cutoff step of cutting off a part of the semiconductor package at a cutoff region provided in a region which does not include the semiconductor chip along a thickness direction of the semiconductor package to form the semiconductor package to be smaller than the semiconductor package before the cutoff.

16. The method for manufacturing a semiconductor device according to claim 15, wherein the semiconductor package is configured such that a region around the semiconductor chip is filled with a sealing material in the storage portion sealed with a cover glass, and the cutoff region is provided in the region filled with the sealing material in a plan view, and
the cutoff step is performed by cutting the semiconductor package at a large-diameter part of a wire which is formed to be thicker at the cutoff region than at another position and is electrically connected to an external connection terminal of the semiconductor chip.

17. The method for manufacturing a semiconductor device according to claim 15, wherein the semiconductor package is configured such that a frame is attached via a second sealing material to a side in the thickness direction of a structure in which a region around the semiconductor chip placed in the storage portion is filled with a first sealing material, and a cover glass is attached to the first sealing material to overlap with the semiconductor chip in a plan view, and the cutoff region is provided at the second sealing material, and
the cutoff step is performed by cutting the second sealing material.

18. The method for manufacturing a semiconductor device according to claim 15, wherein the semiconductor package is configured such that a region around the semiconductor chip is filled with a sealing material in the storage portion sealed with a cover glass, a wire which is electrically connected to an external connection terminal of the semiconductor chip is formed to extend from the external connection terminal in a width direction of the semiconductor package and be substantially perpendicularly bent so as to extend along the thickness direction of the semiconductor package, and the cutoff region is provided outside the wire in the width direction in a plan view at the sealing material, and
the cutoff step is performed by grinding away or cutting a part outside the wire in the width direction of the package at the sealing material in a plan view without cutting the wire.
